Europäisches Patentamt

European Patent Office

Office européen des brevets

(19)

(11) Veröffentlichungsnummer: **0 244 596**

**A1**

(12) ## EUROPÄISCHE PATENTANMELDUNG

(21) Anmeldenummer: 87103505.1

(22) Anmeldetag: 11.03.87

(51) Int. Cl.⁴ **A61B 6/00**

(30) Priorität: 25.03.86 DE 3610070

(43) Veröffentlichungstag der Anmeldung:
11.11.87 Patentblatt 87/46

(84) Benannte Vertragsstaaten:
DE FR

(71) Anmelder: **Siemens Aktiengesellschaft Berlin
und München
Wittelsbacherplatz 2
D-8000 München 2(DE)**

(72) Erfinder: **Iffländer, Helmut, Dipl.-Ing.
Lindenstrasse 6
D-8521 Langensendelbach(DE)**

(54) **Röntgenuntersuchungsgerät mit einem C-Bogen.**

(57) An den Enden eines C-Bogens (1) sind ein Röntgenstrahler (2) und ein Strahlenempfänger (3) einander gegenüberliegend angeordnet. Der C-Bogen (1) ist in Umfangsrichtung verstellbar an einem Halter (5) gelagert. Der Halter (5) ist um eine horizontale Achse (6) schwenkbar mit einem Wagen (7) verbunden. Der Wagen (7) ist an einer vertikalen Säule (8) längsverschiebbar gelagert. Die Säule (8) ist in parallelen Schienen (9, 10) senkrecht zur horizontalen Achse (6) verfahrbar. Der Patientenlagerungstisch (11) ist im Vergleich zur Säule (8) geringfügig in der Verstellrichtung der Säule verschiebbar. um das Positionieren eines Patienten auf dem Patientenlagerungstisch zu erleichtern.

EP 0 244 596 A1

## Röntgenuntersuchungsgerät mit einem C-Bogen

Die Erfindung betrifft ein Röntgenuntersuchungsgerät mit einem C-Bogen, an dessen Enden ein Röntgenstrahler und ein Strahlenempfänger einander gegenüberliegend befestigt sind, welcher einen Patientenlagerungstisch umgreift, wobei die Achse des C-Bogens in einer vertikalen Ebene verläuft, in der auch die Längsachse des Patientenlagerungstisches liegt, der C-Bogen in Umfangsrichtung verstellbar an einem Halter gelagert ist, der Halter um eine horizontale Achse schwenkbar mit einem Wagen verbunden ist, die senkrecht auf der vertikalen Ebene steht, der Wagen an einer vertikalen Säule längsverschiebbar gelagert ist, und die Säule in parallelen Schienen senkrecht zur horizontalen Achse verfahrbar ist.

Mittels derartiger Röntgenuntersuchungsgeräte ist es möglich, alle wesentlichen Einstellbewegungen bei der Durchführung einer Untersuchung durch Verstellen des C-Bogens vorzunehmen. Dies ist insbesondere bei der Angiokardiographie von Bedeutung, wo der Verlauf eines Kontrastmittels im menschlichen Körper während der Untersuchung durch Verschieben des C-Bogens verfolgt wird.

Ein Röntgenuntersuchungsgerät der eingangs genannten Art ist aus der DE-C-968 128 bekannt. Nachteilig an diesem Gerät, dessen Patientenlagerungstisch lediglich höhenverstellbar und um eine horizontale Achse schwenkbar ist, ist, daß dann, wenn bei einer Untersuchung die den C-Bogen tragende Säule ausgehend von einer ihrer beiden Endpositionen, in die sie in den Schienen verfahrbar ist, längs des Patientenlagerungstisches verschoben werden soll, eine exakte Positionierung des Patienten auf dem Patientenlagerungstisch relativ zu dem Röntgenstrahler und dem Strahlenempfänger wenigstens in Längsrichtung des Patienten lagerungstisches erforderlich ist. Diese Positionierung kann aber wenigstens in der einen Richtung nicht durch Verfahren der Säule erfolgen, da sich diese in einer Endposition befindet. Die Positionierung des Patienten auf dem Patientenlagerungstisch muß somit manuell erfolgen, was insbesondere für bewegungsunfähige und hilflose Patienten wegen der dabei von dem Bedienungspersonal ausgeübten Kräfte unangnehm ist oder sogar schmerzhaft sein kann. Für das Bedienpersonal ist die Positionierung des Patienten wegen der damit verbundenen körperlichen Anstrengungen unbequem.

Der Erfindung liegt die Aufgabe zugrunde, ein Röntgenuntersuchungsgerät der eingangs genannten Art so auszubilden, daß es genügt, den Patienten beim Betten auf den Patientenlagerungstisch grob auszurichten, und die exakte Positionierung des Patienten auf für diesen schonende Weise und für das Bedienpersonal ohne körperliche Anstrengungen möglich ist.

Nach der Erfindung wird dies dadurch erreicht, daß der Patientenlagerungstisch in der Verstellrichtung der Säule verschiebbar ist. Es reicht somit aus, wenn der Patient beim Betten auf dem Patientenlagerungstisch grob positioniert wird, da die exakte Positionierung durch Verschieben des Tisches ohne Krafteinwirkung auf den Patienten und ohne körperliche Anstrengung für das Bedienpersonal erfolgen kann. Dabei genügt es übrigens, wenn der Patientenlagerungstisch eine relativ geringfügige Verstellbarkeit in der Größenordnung von etwa 40 cm aufweist. Im Hinblick auf eine leichtere Positionierbarkeit des Patienten kann übrigens auch eine Verstellbarkeit des Patientenlagerungstisches quer zu seiner Längsachse von Vorteil sein.

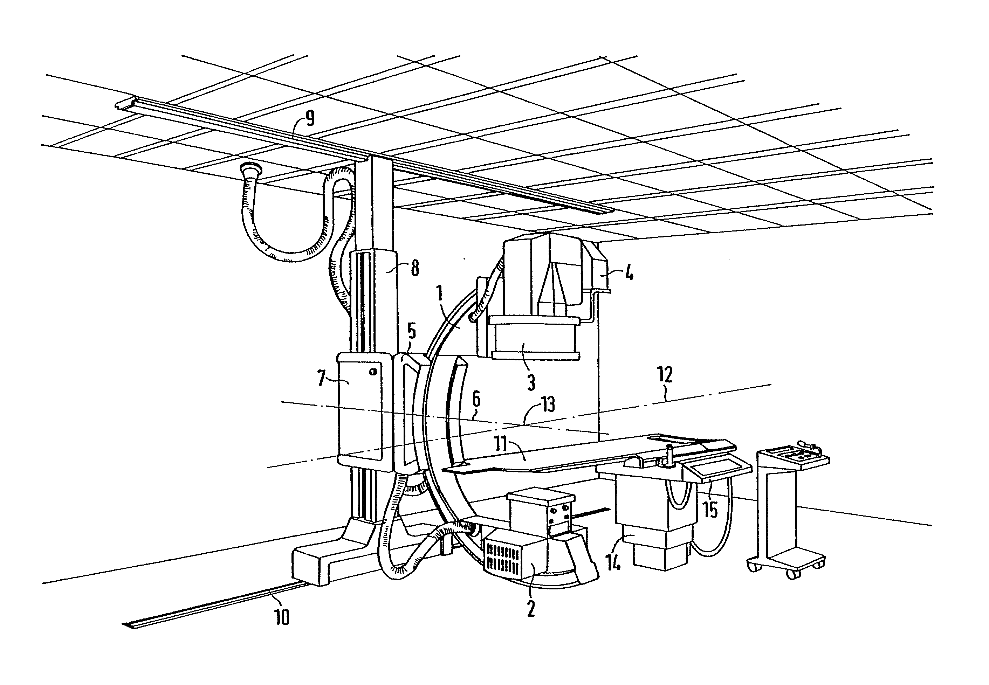

Die Erfindung ist nachfolgend anhand eines in der Zeichnung dargestellten Ausführungsbeispieles näher erläutert.

In der Zeichnung ist ein C-Bogen 1 dargestellt, der an seinen Enden einen Röntgenstrahler 2 und einen Röntgenbildverstärker 3 mit nachgeschalteter Einzelbildkamera 4 sowie nicht sichtbarer Fernsehkamera trägt. Der C-Bogen 1 ist längs seines Umfanges an einem Halter 5 verstellbar gelagert, welcher um eine horizontale Achse 6 schwenkbar mit einem Wagen 7 verbunden ist. Der Wagen 7 ist an einer vertikalen Säule 8 längsverschiebbar gelagert, welche in einer Deckenschiene 9 und einer Bodenschiene 10, welche parallel zueinander und zur Längsrichtung eines Patientenlagerungstisches 11 verlaufen, parallel zur Längsrichtung des Patientenlagerungstisches 11 verfahrbar ist. Der C-Bogen 1 umgreift den Patientenlagerungstisch 11 quer, d.h. seine Achse 12 liegt in einer vertikalen Ebene, in der auch die Längsachse des Patientenlagerungstisches 11 liegt. Die Achsen 6 und 12 schneiden sich im Isozentrum 13. Unter der Achse 12 ist dabei die Achse des C-Bogens zu verstehen, die durch dessen Mittelpunkt geht und auf seiner Ebene senkrecht steht.

Der Patientenlagerungstisch 11 ist auf einem fest am Boden angebrachten Sockel 14 höhenverstellbar gelagert, an dem ein Bedienpult 15 befestigt ist. Er ist gegenüber dem Sockel 14 in Längs-und Querrichtung verstellbar (schwimmende Lagerung), wobei jedoch der Stellweg in Längsrichtung klein ist und in der Größenordnung von 40 cm liegt.

Zur Durchführung einer Untersuchung, bei der die Säule 8 ausgehend von einer ihrer Endpositionen längs des Patientenlagerungstisches verfahren werden soll, wird so vorgegangen, daß zunächst der Patientenlagerungstisch 11 auf seinem Sockel 14 hinsichtlich der Verstellbarkeit in Längsrichtung in eine mittlere Lage gebracht wird, so daß er in beiden Richtungen parallel zu seiner Längsachse verstellbar ist. Anschließend wird der Patient auf den Patientenlagerungstisch 11 gebettet und durch Verstellen des Patientenlagerungstisches 11 in dessen Längsrichtung und erforderlichenfalls in dessen Querrichtung exakt in der gewünschten Weise relativ zu dem Röntgenstrahler 2 und dem Röntgenbildverstärker 3 positioniert. Dieser Positioniervorgang erfolgt infolge der Verstellbarkeit des Patientenlagerungstisches 11 auf für den Patienten schonende Weise, da dazu keine Kräfte auf ihn ausgeübt werden müssen. Nach erfolgter Positionierung des Patienten wird die eigentliche Untersuchung unter Verstellung des C-Bogens 1, z.B. durch Verfahren der Säule 8 in den Schienen 9 und 10 längs des Patientenlagerungstisches 11, durchgeführt.

Zur Veränderung des Abstandes Fokus - Bildschicht ist der Röntgenbildverstärker 3 am C-Bogen 1 in radialer Richtung verstellbar gelagert.

## Ansprüche

Röntgenuntersuchungsgerät mit einem C-Bogen (1), an dessen Enden ein Röntgenstrahler (2) und ein Strahlenempfänger (3) einander gegenüberliegend befestigt sind, welcher einen Patientenlagerungstisch (11) umgreift, wobei die Achse (12) des C-Bogens (1) in einer vertikalen Ebene verläuft, in der auch die Längsachse des Patientenlagerungstisches (11) liegt, der C-Bogen (1) in Umfangsrichtung verstellbar an einem Halter (5) gelagert ist, der Halter (5) um eine horizontale Achse (6) schwenkbar mit einem Wagen (7) verbunden ist, die senkrecht auf der vertikalen Ebene steht, der Wagen (7) an einer vertikalen Säule (8) längsverschiebbar gelagert ist, und die Säule (8) in parallelen Schienen (9, 10) senkrecht zur horizontalen Achse (6) verfahrbar ist, **dadurch gekennzeichnet,** daß der Patientenlagerungstisch (11) in der Verstellrichtung der Säule (8) verschiebbar ist.

| Europäisches Patentamt | **EUROPÄISCHER RECHERCHENBERICHT** | Nummer der Anmeldung |
|---|---|---|
| | | EP 87 10 3505 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,A | DE-A-1 466 880 (PICKER CORP.) * Figuren 1,2; Seite 12, Zeile 21 - Seite 16, Zeile 9; Seite 18, Zeile 4 - Seite 26, Zeile 9 * | 1 | A 61 B 6/00 |
| | --- | | |
| Y | US-A-4 024 401 (BERNSTEIN) * Figur 2; Spalte 3, Zeilen 25-48; Spalte 4, Zeilen 9-54 * | 1 | |
| | --- | | |
| D,A | DE-C- 968 128 (KOCH UND STERZEL AG) * Figuren 1,2; Seite 2, Zeile 83 - Seite 3, Zeile 25 * | 1 | |
| | ----- | | |

RECHERCHIERTE
SACHGEBIETE (Int. Cl.4)

A 61 B

Der vorliegende Recherchenbericht wurde für alle Patentansprüche erstellt.

| Recherchenort | Abschlußdatum der Recherche | Prüfer |
|---|---|---|
| DEN HAAG | 14-07-1987 | CHEN A.H. |